Europäisches Patentamt

European Patent Office

Office européen des brevets

(11) Veröffentlichungsnummer : **0 037 015**
**B1**

(12) **EUROPÄISCHE PATENTSCHRIFT**

(45) Veröffentlichungstag der Patentschrift :
22.06.83

(21) Anmeldenummer : 81102029.6

(22) Anmeldetag : 18.03.81

(51) Int. Cl.³ : **C 07 C 69/716**, C 07 C 67/46,
C 07 C 67/14

(54) Verfahren zur Herstellung von 2-Chloracetessigsäureestern.

(30) Priorität : 02.04.80 CH 2606/80

(43) Veröffentlichungstag der Anmeldung :
07.10.81 Patentblatt 81/40

(45) Bekanntmachung des Hinweises auf die Patenterteilung : 22.06.83 Patentblatt 83/25

(84) Benannte Vertragsstaaten :
**AT BE CH DE FR GB IT LI NL SE**

(56) Entgegenhaltungen :
US A 2 209 683
US A 3 666 793
CHEMICAL ABSTRACTS, Band 86, Nr. 19, 09-05-1977, Seite 506, Zusammenfassung 139408h Columbus, Ohio, US
CHEMICAL ABSTRACTS, Band 34, Nr. 18, 20-09-1940, Spalte 6222B Columbus, Ohio, US
C. D. HURD et al. : « Chlorination and structure of acetylketene »
CHEMICAL ABSTRACTS, Band 46, Nr. 17, 10-09-1952, Spalte 8115 Columbus, Ohio, US
V. V. PEREKALIN et al. : « Reactions of diketene. Reaction of diketene with some weakly basic aromatic amines »
CHEMISCHE BERICHTE, Band 81, Nr. 5 Oktober 1948, Berlin, DE H. BRINTZINGER et al. : « Notiz über Thioketone », Seiten 380, 381

(73) Patentinhaber : **LONZA AG**

**Gampel/Wallis (CH)**

(72) Erfinder : **Blum, René, Dr.**
**Bollwerkstrasse 60**
**CH-Binningen (Kanton Baselland) (CH)**
Erfinder : **Tenud, Leander, Dr.**
**Balfrinstrasse 23**
**CH-Visp (Kanton Wallis) (CH)**

(74) Vertreter : **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Siegfriedstr. 8**
**D-8000 München 40 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Herstellung von 2-Chloracetessigsäureestern

Die vorliegende Erfindung betrifft ein Verfahren zur Herstellung von 2-Chloracetessigsäureestern. 2-Chloracetessigsäureester können zur Herstellung von Cumarilsäurederivaten verwendet werden, die als Zwischenprodukt zur Herstellung von Pflanzenschutzmitteln bekannt sind. 2-Chloracetessigsäureester stellen auch Zwischenprodukte für pharmazeutische Präparate, z. B. Cimetidin, dar.

Bei den bisher bekannten Herstellungsverfahren wird von Acetessigsäureestern ausgegangen, die entweder mit Sulfurylchlorid oder mit Chlor chloriert werden.

Die Chlorierung mit Sulfurylchlorid verläuft zwar bis zu hohen Umsätzen mit guter Ausbeute (93-97 %), hat jedoch den Nachteil, dass die bei der Reaktion entstehenden Gase, $SO_2$ und HCl, getrennt werden müssen und das Schwefeldioxid in einem speziellen Arbeitsprozess wieder in das Sulfurylchlorid umgewandelt werden muss.

In der DE-OS 27 09 138 wird ein Verfahren beschrieben, bei dem 2-Chloracetessigsäureester durch Chlorierung von Acetessigsäureestern hergestellt wird. Die dabei erhaltenen Ausbeuten an dem gewünschten Produkt sind bei diskontinuierlicher Arbeitsweise vergleichsweise niedrig. Bei Anwendung eines kontinuierlichen Verfahrens werden zwar höhere Ausbeuten erhalten, es ist jedoch die Verwendung eines Silberkühlers oder eines Fallfilmreaktors vorgesehen. Des weiteren ist es außerordentlich wünschenswert, die Herstellung von 2-Chloracetessigsäureestern ausgehend von Diketen in einem « Eintopfverfahren » durchzuführen, wobei trotzdem ausgezeichnete Ausbeuten, und zwar bezogen auf das eingesetzte Diketen, erhalten werden sollen.

Ziel der vorliegenden Erfindung ist ein einfaches, leistungsfähiges Verfahren. Erfindungsgemäss wird dies dadurch erreicht, dass man von Diketen ausgeht, dieses bei Temperaturen von + 10 bis − 40 °C in Gegenwart von chloriertem aliphatischen Kohlenwasserstoff vorzugsweise mit 1 bis 3 Kohlenstoffatomen als Lösungsmittel mit HCl in das Acetessigsäurechlorid überführt, durch Einleiten von Chlor bei Temperaturen von + 10 bis − 40 °C das 2-Chloracetessigsäurechlorid bildet und letzteres durch Zugabe von vorzugsweise aliphatischen Alkoholen mit 1 bis 4 C-Atomen in den entsprechenden Ester überführt.

Die Chlorierung kann in Gegenwart von sauren Katalysatoren, wie Lewis-Säuren, z. B. $PCl_3$, $FeCl_3$, anorganischen Säuren, z. B. Schwefelsäure, Perchlorsäure, oder starken organischen Carbonsäuren, z. B. Essigsäure, Trifluoressigsäure, p-Toluolsulfonsäure, durchgeführt werden. Diese Säuren werden in katalytischen Mengen, zweckmässig 0,1 bis 20 Mol.%, bezogen auf eingesetztes Diketen, zugesetzt.

Als Lösungsmittel werden chlorierte aliphatische Kohlenwasserstoffe, wie Tetrachlorkohlenstoff, Aethylchlorid, Dichloräthan usw., vorzugsweise Methylenchlorid, verwendet. Zweckmässig setzt man pro Mol Diketen 200 bis 1 000 g Lösungsmittel ein. Das Molverhältnis HCl zu Diketen in der ersten Stufe kann stark variieren, zweckmässig wird pro Mol Diketen 1,1 bis 10 Mol, vorzugsweise 1,95 bis 6,5 Mol HCl, angewendet.

Für die Chlorierung mit Chlor in der zweiten Stufe werden vorzugsweise pro Mol Acetessigsäurechlorid 0,9 bis 1,1 Mol Chlor angewendet.

Zur Herstellung der gewünschten Ester wird das in der zweiten Stufe erhaltene 2-Chloracetessigsäurechlorid mit dem entsprechenden Alkohol mit 1 bis 4 C-Atomen in einer dritten Stufe umgesetzt. Solche Alkohole sind Methyl-, Aethyl-, Propyl-, Butylalkohol.

Die hinzuzusetzende Menge an Alkohol sind die stöchiometrischen Mengen. Es kann in manchen Fällen zweckmässig sein, einen leichten Ueberschuss, zweckmässig von 0,1 bis 0,3 Mol, anzuwenden.

Eine besonders bevorzugte Ausführungsform der Erfindung besteht darin, dass man die drei Stufen im selben Reaktionsmedium und Reaktionsgefäss, ohne dass eines der Zwischenprodukte isoliert werden muss, durchführen kann.

### Beispiel 1

In einem Doppelmantelkolben von 500 ml Grösse, ausgestattet mit Pressluftrührer, Thermometer, Gaseinleitungsrohr mit Fritte, Rückflusskühler und Kryomat, wurden 50,45 g (0,6 Mol) Diketen in 300 ml Methylenchlorid gelöst und auf − 20 °C abgekühlt. Nach Zusatz von 0,11 Mol.% $H_2SO_4$, bezogen auf Diketen, und 43,8 g (1,2 Mol) Chlorwasserstoff, bezogen auf Diketen, wurden nach 3 1/2 Stunden Rühren während 35 Minuten 42,5 g (0,6 Mol) Chlor eingeleitet und weitere 30 Minuten gerührt. Anschliessend wurde immer noch bei − 20 bis − 10 °C 27,6 g (0,6 Mol) absoluter Aethanol zugetropft und während 15 Minuten gerührt.

Anschliessend wurde das Lösungsmittel am Rotavap abgedampft. Es resultierten 98,7 g 2-Chloracetessigsäureäthylester mit einem Gehalt von 83,4 Gew.%.

### Beispiele 2-6

Nachstehende Tabelle zeigt die Ergebnisse weiterer Versuche.

(Siehe die Tabelle, Seite 3)

2

| | Katalysator und %, bezogen auf Diketen | Lösungs-mittel | Mol-Verhält-nis HCl/Diketen | Tempera-tur °C | Ausbeute in % 2-Chloracet-essigester bez. auf Diketen |
|---|---|---|---|---|---|
| 2 | 0,1 $H_2SO_4$ | $CH_2Cl_2$ 300 ml | 1,95 | − 20 | 88,4 |
| 3 | 0,1 $H_2SO_4$ | $CH_2Cl_2$ 300 ml | 1,95 | 0 | 86,0 |
| 4 | — | $CH_2Cl_2$ 300 ml | 2,00 | 0 | 83,0 |
| 5 | 0,1 $CH_3COOH$ | $CCl_4$ 300 ml | 6,3 | − 40 | 83,0 |
| 6 | 10,0 $PCl_3$ | $CH_2Cl_2$ 300 ml | 2,1 | − 20 | 87,4 |

**Ansprüche**

1. Verfahren zur Herstellung von 2-Chloracetessigsäureestern, dadurch gekennzeichnet, dass man Diketen bei Temperaturen von + 10 bis − 40 °C in Gegenwart von chlorierten aliphatischen Kohlenwasserstoffen als Lösungsmittel mit Hilfe von Chlorwasserstoff in das Acetessigsäurechlorid überführt, durch Einleiten von Chlor bei Temperaturen von + 10 bis − 40 °C das 2-Chloracetessigsäurechlorid bildet und letzteres durch Zugabe von Alkoholen mit 1 bis 4 C-Atomen bei Temperaturen von + 10 bis − 40 °C in den entsprechenden Ester überführt.

2. Verfahren nach Anspruch 1, dadurch gekennzeichnet, dass man die Chlorierung in Gegenwart von sauren Katalysatoren durchführt.

3. Verfahren nach Ansprüchen 1 und 2, dadurch gekennzeichnet, dass man als Lösungsmittel Methylenchlorid verwendet.

4. Verfahren nach einem oder mehreren der Ansprüche 1-3, dadurch gekennzeichnet, daß man die Dreistufenreaktion ohne Isolierung der Zwischenproduckte durchführt.

**Claims**

1. Process for the production of 2-chloroacetoacetic esters, characterized in converting diketene at temperatures of from + 10 to − 40 °C in the presence of chlorinated aliphatic hydrocarbons as solvent by using hydrogen chloride into the acetoacetic chloride, forming the 2-chloroacetoacetic chloride by introducing of chlorine at temperatures of from + 10 to − 40 °C, and converting the 2-chloroacetoacetic chloride by adding of alcohols having from 1 to 4 carbon atoms at temperatures of from + 10 to − 40 °C into the corresponding esters.

2. Process according to claim 1, characterized in that the chlorination is carried out in the presence of acidic catalysts.

3. Process according to claims 1 and 2, characterized in using methylene chloride as solvent.

4. Process according to one or more of the claims 1-3, characterized in that the three-step-reaction is carried out without isolating the intermediate products.

**Revendications**

1. Procédé de préparation d'esters de l'acide 2-chloroacétoacétique, caractérisé en ce que l'on transforme du dicétène en le chlorure de l'acide acétoacétique à des températures de + 10 à − 40 °C en présence d'hydrocarbures aliphatiques chlorés en tant que solvants à l'aide de chlorure d'hydrogène, on forme le chlorure de l'acide 2-chloroacétoacétique par introduction de chlore à des températures + 10 à − 40 °C et on transforme ce dernier en l'ester correspondant par addition d'alcools avec 1 à 4 atomes de C à des températures de + 10 à − 40 °C.

2. Procédé selon la revendication 1, caractérisé en ce que la chloration est effectuée en présence de catalyseurs acides.

3. Procédé selon les revendications 1 et 2, caractérisé en ce que l'on utilise en tant que solvant du chlorure de méthylène.

4. Procédé selon l'une ou plusieurs des revendications 1-3, caractérisé en ce que l'on effectue la réaction en trois étapes sans isolement des produits intermédiaires.